# FASCICULE DE BREVET EUROPEEN

(11) **EP 1 304 098 B1**
(45) Date de publication et mention de la délivrance du brevet: **11.11.2009**
(21) Numéro de dépôt: 02292242.1
(22) Date de dépôt: 12.09.2002
(51) Int. Cl.: A61K 8/00, A61K 8/02, A61K 8/06, A61K 8/91, A61K 8/92, A61Q 1/00, A61Q 1/02, A61Q 1/14, A61Q 5/00, A61Q 5/12, A61Q 19/00, B01F 17/54, C08L 83/10

(54) **D'émulsion huile-dans-eau contenant un copolymère siliconé et utilisations cosmétiques**
Öl-in-Wasser Emulsion, die ein Siliconcopolymer enthält, und kosmetische Verwendungen
Oil -in-water emulsion containing a silicone copolymer and cosmetic uses

(30) Priorité: 15.10.2001 FR 0113272
(43) Date de publication de la demande: 23.04.2003
(73) Titulaire: L'ORÉAL, 75008 Paris (FR)
(72) Inventeur: Lennon, Paula, 69003 Lyon (FR)
(74) Mandataire: Rasson, Catherine

(56) Documents cités:
- EP-A- 1 097 703
- WO-A-95/28913
- WO-A-97/32560
- WO-A-97/32561
- US-A- 6 013 682

## Description

La présente demande se rapporte à une composition sous forme d'émulsion huile-dans-eau, comprenant une phase huileuse dispersée dans une phase aqueuse, la dite phase aqueuse comprenant des particules d'un copolymère siliconé, et à l'utilisation de la dite composition dans les domaines cosmétique et dermatologique, en particulier pour le soin et/ou le traitement de la peau du corps ou du visage, des cheveux et/ou des lèvres.

Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme une phase externe, une phase aqueuse, ce qui leur confère, lors de l'application sur la peau, un toucher plus frais, moins gras et plus léger que les émulsions E/H.

Par ailleurs, il est connu que les tensioactifs susceptibles de former des phases lamellaires anisotropes (cristaux liquides) permettent d'obtenir des émulsions stables particulièrement avantageuses : voir à ce sujet G. DAHMS, Cosmetics & Toiletries, 1986, vol.101, p. 113-115. Toutefois, de telles émulsions présentent l'inconvénient d'avoir des propriétés cosmétiques insuffisantes (toucher huileux, frein à l'application et manque de douceur).

Généralement, ces émulsions contiennent en outre un agent épaississant dont la fonction est de créer, au sein de la phase aqueuse, une matrice gélifiée servant à figer les gouttelettes huileuses et assurant un maintien mécanique de l'ensemble de l'émulsion. Toutefois, l'addition de tels agents présente l'inconvénient de ne pas permettre d'obtenir toutes les textures désirées et en particulier les textures fluides, légères, s'appliquant facilement et rapidement sur la peau sans laisser de film résiduel.

L'objectif de l'invention est de pouvoir réaliser des émulsions huile-dans-eau stables, présentant de bonnes propriétés cosmétiques sans avoir les inconvénients de l'art antérieur, et ce, quelle que soit la texture souhaitée et notamment quelle que soit la viscosité de l'émulsion.

La demanderesse a découvert de façon inattendue une nouvelle famille de polymères permettant de réaliser des émulsions huile-dans-eau stables et ayant de très bonnes propriétés cosmétiques, quelle que soit la texture envisagée.

La présente invention concerne une composition sous forme d'émulsion huile-dans-eau comprenant dans un milieu physiologiquement acceptable, une phase huileuse dispersée dans une phase aqueuse, caractérisée par le fait que la phase aqueuse comprend des particules de copolymère siliconé non réticulé et au moins un tensioactif amphiphile susceptible de former des cristaux liquides, par le fait que la phase huileuse contient au moins une huile, et par le fait que le copolymère siliconé est obtenu par réaction d'extension de chaîne, en présence d'un catalyseur, à partir d'au moins :
- (a) un polysiloxane (i) qui est le diméthylvinyl-siloxy-polydiméthylsiloxane; et
- (b) un composé organosiliconé (ii) qui réagit avec le polysiloxane (i) par réaction d'extension de chaîne, et qui est un organohydrogenopolysiloxane liquide de formule (II) telle que définie ci-dessous.

La composition de l'invention constitue notamment une composition à application topique, en particulier cosmétique ou dermatologique.

On entend dans la présente demande « tensioactif amphiphile susceptible de former des cristaux liquides » tout tensioactif capable de donner lieu à la formation de cristaux liquides entre 20 et 40°C soit dans la composition elle-même soit lors de l'application de la composition sur la matière kératinique et notamment sur la peau. On peut trouver des exemples de ces phases cristal liquide dans l'ouvrage « Advances in Liquid Crysals », vol. 1, Edition Glenn H. BROWN, 1995, pages 1 à 139.

La formation de cristaux liquides peut être mise en évidence par microscopie optique en polarisation croisée, éventuellement associée à la diffraction des Rayons X.

Les cristaux liquides de la présente invention sont de type lyotrope lamellaire ou hexagonal ou cubique, direct ou inverse.

Certes, une émulsion H/E contenant une suspension aqueuse de particules d'organosiloxane élastomère réticulé est connu par le document EP-A-1,097,703. Toutefois, l'organosiloxane décrit dans ce document est un élastomère réticulé alors que, dans la présente demande, le copolymère siliconé n'est ni élastomère ni réticulé, mais est un copolymère bloc, ce que n'est pas l'organosiloxane réticulé du document EP-A-1,097,703. En outre, la composition selon l'invention présente de manière surprenante l'avantage sur cet art antérieur d'être stable sans avoir à ajouter d'agents épaississants et donc d'être dépourvue des inconvénients indiqués ci-dessus. En outre, les propriétés cosmétiques des compositions selon l'invention sont meilleures que celles des compositions contenant des particules d'élastomère de silicone, en particulier les compositions de l'invention présentent une plus grande douceur et ont moins tendance à faire des peluches lors de l'application sur la peau.

Par ailleurs, le document WO-A-97/32561 décrit des émulsions H/E contenant une phase siliconée comprenant un polyorganosiloxane élastomère. Toutefois, le polyorganosiloxane utilisé dans ce document est élastomère et donc réticulé contrairement au copolymère siliconé de la présente demande. Par ailleurs, il n'est pas en suspension aqueuse mais en milieu huileux, et il est introduit dans la phase huileuse et notamment siliconée de l'émulsion et non dans la phase aqueuse, ce qui a pour inconvénient de devoir suivre, pour la préparation des compositions contenant ce type de composés, un mode opératoire bien spécifique et contraignant, ce qui n'est pas le cas pour le copolymère utilisé dans la présente demande. En outre, quand le polyorganosiloxane est dans la phase huileuse, la compatibilité avec les autres constituants est aléatoire, notamment quand il s'agit d'huile de silicone, et la stabilité non assurée.

La composition obtenue selon l'invention a une texture homogène et agréable à l'application. En outre, la dispersion de particules de copolymère siliconé utilisée dans la composition selon l'invention permet de préparer des émulsions huile-dans-eau, qui restent stables dans le temps à température ambiante ou à des températures plus élevées, et de conserver ces propriétés de l'émulsion, quelle que soit la fluidité de l'émulsion. On peut donc ainsi préparer aussi bien des émulsions épaisses particulièrement efficaces pour le traitement des peaux sèches que des émulsions très fluides. La viscosité des émulsions peut donc varier dans une large mesure et aller par exemple de 0,05 Pa.s à 20 Pa.s, de préférence de 0,05 à 10 Pa.s, ces viscosités étant mesurées à environ 25°C à l'aide du viscosimètre "Rhéomat 180" qui est, de manière générale, équipé d'un mobile 2 pour les gammes de viscosités de 0,02 Pa.s à 0,7 Pa.s, d'un mobile 3 pour les gammes de viscosités de 0,2 Pa.s à 4 Pa.s, et d'un mobile 4 pour les gammes de viscosités de 2 Pa.s à 23 Pa.s.

La composition de l'invention se présente sous forme d'une émulsion H/E. Etant destinée à une application topique, elle comprend un milieu physiologiquement acceptable, c'est-à-dire un milieu compatible avec toutes les matières kératiniques telles que la peau, les ongles, les muqueuses et les cheveux ou toute autre zone cutanée du corps.

La composition peut comprendre des particules d'une ou plusieurs sortes de copolymères siliconés. Les particules de copolymère siliconé sont présentes dans la composition de l'invention dans des concentrations en matière active pouvant varier largement selon la concentration en huile et selon la viscosité souhaitée. La concentration en matière active de particules de copolymère siliconé va de préférence de 0,01 à 15 % en poids, mieux de 0,1 à 10 % et encore mieux de 0,5 à 5 % en poids par rapport au poids total de la composition.

La taille des particules de copolymère siliconé peut varier largement. De manière préférée dans la présente demande, les particules de copolymère siliconé présentent généralement une taille moyenne en nombre inférieure ou égale à 2 microns, et de préférence inférieure ou égale à 1 micron.

Les particules de copolymères siliconés utilisées selon l'invention peuvent être choisies notamment parmi celles décrites dans le document US-A-6 013 682. Selon ce document, on peut notamment obtenir les copolymères siliconés de ces particules par réaction d'extension de chaîne, en présence d'un catalyseur, à partir d'au moins :
- (a) un polysiloxane (i) ayant au moins un groupe réactif et de préférence un ou deux groupes réactifs par molécule ; et
- (b) un composé organosiliconé (ii) qui réagit avec le polysiloxane (i) par réaction d'extension de chaîne.

Le polysiloxane (i) incorporé dans la composition selon l'invention est choisi parmi les composés de formule (I) 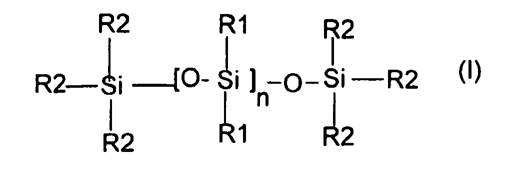 dans laquelle
n est un nombre entier supérieur à 1, et est de fait
le diméthylvinyl-siloxy-polydiméthylsiloxane, compose de formule (I) dans laquelle les radicaux R₁ sont des radicaux méthyle, et, en bout de chaîne, le radical R₂ est un radical vinyle tandis que les deux autres radicaux R₂ sont des radicaux méthyle

Le composé organosiliconé (ii) est un organohydrogenopolysiloxane liquide de formule (II) : 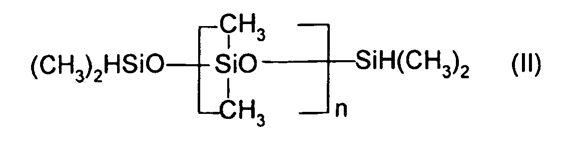 où n est un nombre entier supérieur à 1 et de préférence supérieur à 10, et par exemple allant de 10 à 30. Selon un mode particulier de réalisation de l'invention, n est égal à 20.

Le catalyseur de la réaction entre le polysiloxane et le composé organosiliconé peut être choisi parmi les métaux et notamment parmi le platine, le rhodium, l'étain, le titane, le cuivre et le plomb. Il s'agit de préférence du platine ou du rhodium.

Les particules de copolymère siliconé utilisées selon l'invention se présentent généralement sous forme d'une dispersion aqueuse de particules, qui peut être notamment obtenue par exemple par mélange de (a) l'eau, (b) au moins un émulsifiant, (c) le polysiloxane (i), (d) le composé organosiliconé (ii) et (e) un catalyseur. De préférence, l'un des constituants (c), (d) ou (e) est ajouté en dernier dans le mélange, afin que la réaction d'extension de chaîne ne commence que dans la dispersion.

Comme émulsifiants susceptibles d'être utilisés dans le procédé de préparation décrit ci-dessus pour obtenir la dispersion aqueuse de particules, on peut citer les émulsifiants non ioniques ou ioniques (anioniques, cationiques ou amphotères). Il s'agit de préférence d'émulsifiants non ioniques qui peuvent être choisis parmi les polyalkylène glycols éthers d'alcool gras, comportant de 8 à 30 atomes de carbone et de préférence de 10 à 22 atomes de carbone ; les alkylesters de sorbitan polyoxyalkylénés et notamment polyoxyéthylénés, où le radical alkyle comporte de 8 à 30 atomes de carbone et de préférence de 10 à 22 atomes de carbone ; les alkylesters polyoxyalkylénés et notamment polyoxyéthylénés, où le radical alkyle comporte de 8 à 30 atomes de carbone et de préférence de 10 à 22 atomes de carbone ; les polyéthylène glycols ; les polypropylène glycols ; les diéthylène glycols ; et leurs mélanges. La quantité d'émulsifiant(s) est généralement de 1 à 30 % en poids par rapport au poids total du mélange de réaction.

L'émulsifiant utilisé pour obtenir la dispersion aqueuse de particules est de préférence choisi parmi les polyéthylène glycols éthers d'alcools gras et leurs mélanges, et notamment les polyéthylène glycols éthers d'alcools comportant 12 ou 13 atomes de carbone et de 2 à 100 motifs oxyéthylénés et de préférence de 3 à 50 motifs oxyéthylénés, et leurs mélanges. On peut citer par exemple le C₁₂-C₁₃ Pareth-3, le C₁₂-C₁₃ Pareth-23 et leurs mélanges.

Dans l'invention, les particules de copolymère siliconé sont obtenues à partir de diméthylvinyl-siloxy-polydiméthylsiloxane (ou divinyldimethicone) comme composé (i), et du composé de formule (II) comme composé (ii), de préférence en présence d'un catalyseur de type platine, et la dispersion de particules est de préférence obtenue en présence de C₁₂-C₁₃ Pareth-3 et C₁₂-C₁₃ Pareth-23 comme émulsifiants.

Comme particules de copolymère siliconé, on peut utiliser notamment le produit commercialisé sous la dénomination HMW 2220 par la société Dow Corning (nom CTFA : divinyldimethicone / dimethicone / C₁₂-C₁₃ Pareth-3 / C₁₂-C₁₃ Pareth-23).

La composition de l'invention comprend au moins un tensioactif susceptible de former des cristaux liquides. La quantité de tensioactif(s) susceptible(s) de former des cristaux liquides peut aller par exemple de 0,05 à 20 % en poids de matière active et mieux de 0,1 à 10 % en poids de matière active par rapport au poids total de la composition.

Ces tensioactifs peuvent être choisis parmi les tensioactifs anioniques, cationiques, amphotères, non ioniques et leurs mélanges. Ils peuvent être liquides, semi-solides ou cireux, dispersibles dans l'eau ou dans l'huile.

On utilise de préférence un tensioactif ayant une valeur de HLB (Hydrophilic Lipophilic balance) allant de 2 à 12, et préférentiellement de 2 à 10.

Les tensioactifs amphiphiles utilisés dans la composition de l'invention comportent une partie lipophile et une partie hydrophile.

La partie lipophile comprend au moins une chaîne saturée ou insaturée, linéaire ou ramifiée, ayant de 8 à 30 et de préférence de 10 à 28 atomes de carbone, tels que les chaînes oléique, lanolique, tétradécylique, hexadécylique, isostéarylique, laurique, stéarique, acide d'huile de coco (coconut), ou alkyl- phénylique.

Quand la partie hydrophile est un groupe non ionique, elle peut comporter un groupe choisi parmi les chaînes oxyéthylénées (généralement de 2 à 50 groupes oxyéthylénés), les groupes polyglycérolés, les groupes polyols, les groupes polyols oxalkylénés et par exemple un groupe sucre ou sorbitol oxyalkyléné et notamment oxyéthyléné, et leurs mélanges. Quand la partie hydrophile est anionique, celle-ci peut comporter un groupe choisi parmi le groupement phosphate, le groupement phosphatidylcholine et leurs mélanges.

Les tensioactifs amphiphiles peuvent être par exemple choisis parmi les suivants :
Tensioactifs non ioniques :
   - Alkyl éthers de polyglycérol, linéaires ou ramifiés, comme par exemple le Polyglyceryl-2 oleyl ether, le Polyglyceryl-4 oleyl ether.
   - Alkyl phénols éthoxylés ayant une chaîne alkyle comportant de 8 à 26 atomes de carbone, par exemple les alkyl phénols éthoxylés ayant une chaîne alkyle comportant 9 atomes de carbone (nom CTFA : Nonoxynol), tel que le Nonoxynol-2 comme le produit commercialisé sous la dénomination Igepal CO-210 par la société Rhône Poulenc, et leurs mélanges.
   - Esters de polyols, dérivés d'acides gras comportant de 8 à 30 atomes de carbone, et leurs dérivés oxyalkylénés et notamment oxyéthylénés, les polyols étant préférentiellement choisis parmi les sucres, les C₂-C₆-alkylène glycols, le glycérol, les polyglycérols, le sorbitol, le sorbitan, les polyéthylèneglycols, les polypropylène glycols et leurs mélanges. Les esters de polyols oxyalkylénés peuvent comporter par exemple de 1 à 20 groupes oxyalkylénés et notamment 1 à 20 groupes oxyéthylénés.
      Comme esters de glycérol, on peut citer les monoglycérides tels que monooléine (oléate de glycéryle) ; monolinoléine (linoléate de glycéryle) ; monolaurine (laurate de glycéryle) et leurs mélanges.
      Comme esters de polyglycérol, on peut citer le monoisostéarate de diglycéryle, l'oléate de diglycéryle, le mono-oléate de triglycéryle, le distéarate de diglycéryle, le tristéarate de pentaglyceryle, et leurs mélanges.
      Comme ester de glycérol oxyéthyléné, on peut citer par exemple le stéarate de glycérol oxyéthyléné comportant 20 motifs oxyéthylénés, comme le produit commercialisé sous la dénomination Tagat S par la société Goldschmidt.
      Comme esters de sorbitan, on peut citer par exemple le stéarate de sorbitan tel que le produit commercialisé sous la dénomination Span 60 par la société ICI, le laurate de sorbitan tel que le produit commercialisé sous la dénomination Span 20 par la société ICI, le palmitate de sorbitan tel que le produit commercialisé sous la dénomination Span 40 par la société ICI, et le tristéarate de sorbitan tel que le produit commercialisé sous la dénomination Span 65 par la société ICI, l'oléate de sorbitan tel que le produit commercialisé sous la dénomination Span 80 par la société ICI, le trioléate de sorbitan tel que le produit commercialisé sous la dénomination Span 85 par la société ICI. Comme ester de sorbitan oxyéthyléné, on peut citer les Polysorbate et par exemple le Polysorbate 21 commercialisé sous la dénomination Tween 40 par la société ICI, et leurs mélanges.
      Comme esters de sucre, on peut citer ceux dérivés des sucres suivants : sucrose, glucose, fructose, mannose, galactose, arabinose, xylose, maltose, cellibiose, lactose, tréhalose, raffinose, gentianose. On peut citer par exemple le cocoate de sucrose, le monooctanoate de sucrose, le monodecanoate de sucrose, le monolaurate de sucrose, le monomyristate de sucrose, le monopalmitate de sucrose, le monostéarate de sucrose, le monooléate de sucrose, le monolinoléate de sucrose, le dioléate de sucrose, le dipalmitate de sucrose, le distéarate de sucrose, le dilaurate de sucrose, le dilinoléate de sucrose, le tristéarate de sucrose, les esters de β octyl glucofuranoside, les Galactolipides commercialisés par la société Scotia Lipid Teknik, et leurs mélanges.
   - Ethers de polyol, dérivés d'alcools comportant de 8 à 30 atomes de carbone, et notamment les éthers de sucre, tels que les éthers de glucose comme notamment les alkyl-polyglucoside (APG) comme le décylglucoside (Alkyl-C9/C11-polyglucoside (1.4)) tel que le produit commercialisé sous la dénomination MYDOL 10 par la société Kao Chemicals, ou sous la dénomination PLANTAREN 2000 UP par la société Henkel ; le caprylyl/capryl glucoside comme le produit commercialisé sous la dénomination ORAMIX CG 110 par la Société Seppic ; le cétostéaryl glucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Cognis, ainsi que l'arachidyl glucoside, par exemple sous la forme d'un mélange avec les alcools arachidique et béhénique, mélange commercialisé sous la dénomination Montanov 202 par la société Seppic, et leurs mélanges.
   - Silicones oxyéthylénés tels que le produit commercialisé sous la dénomination DC2-5695 par la société Dow Corning.
   - Polymères blocs d'oxyde d'éthylène et d'oxyde de propylène, notamment les Poloxamers tel que le Poloxamer 231 commercialisé sous la dénomination Synperonic PE/L81 par la société Uniqema.
   - Et leurs mélanges.
Tensioactifs anioniques
   - Acides et sels d'acides carboxyliques comportant de 8 à 22 atomes de carbone, de préférence de 8 à 18 atomes de carbone, tels que l'oléate de potassium, le laurate de potassium, le 10-undecenoate de potassium, le myristate de potassium, le palmitate de potassium et le stéarate de potassium ;
   - Esters et sels d'acide phosphonique ou d'acide phosphorique, tels que les phospholipides, naturels et synthétiques, phosphoglycérides, glycolipides, sphingolipides par exemple les céramides, la lécithine, la lysolécithine ;
   - Diesters d'acide phosphorique, tels que le dioleyl phosphate de sodium ;
   - Alkane sulfonates et leurs sels, tels que le 4-dodecyl benzene sulfonate ;
   - Sulfosuccinates tels que le dibutyl sulfosuccinate de sodium ou le di-2 ethylhexyl sulfosuccinate de sodium.
   - Alkyl sulfates et leurs sels, tels que le lauryl sulfate et ses sels ;
   - Dérivés de la phosphotidylcholine tel que le diolylphosphatidylcholine ;
   - Et leurs mélanges.
Tensioactifs cationigues
   - Dérivés quaternaires d'alkyl-imidazolines tels que les chlorures d'alkyl hydroxyéthyl imidazolinium et notamment le chlorure de stéaryl hydroxyéthyl imidazolinium ;
   - Amines éthoxylées telles que les alkylamines oxyéthylénées ayant une chaîne alkyle comportant au moins 14 atomes de carbone ;
   - Alkylamines telles que les diméthyl alkylamines et leurs dérivés, par exemple le produit commercialisé sous la dénomination Armeen 18D par la société Akso Nobel, ou les dialkyl diméthyl amines et leurs dérivés tels que le bromure de didodecyldimethylammonium ;
   - Dérivés quaternaires d'alkylbenzyle, tels que le chlorure de benzalkonium ;
   - Et leurs mélanges.
Tensioactifs amphotères et zwitterioniques :
   - Alkyl betaïnes telles que l'oleyl betaïne ;
   - Alkylamido propyl bétaines telles que la cocamide propyl bétaine commercialisée sous la dénomination LEBON 2000 HG par la société Sanyo ;
   - Et leurs mélanges.

On peut aussi utiliser un mélange d'un ou plusieurs tensioactifs de différentes catégories (anionique, cationique, non ionique, amphotère ou zwitterionique).

Selon un mode préféré de réalisation de l'invention, le tensioactif amphiphile est choisi parmi les tensioactifs non ioniques, plus particulièrement parmi les esters et éthers de polyol, les esters de polyol oxyalkylénés, ou encore mieux parmi les esters de sorbitan, les esters de sucre, les éthers de sucre, et leurs mélanges.

Selon un mode particulier de réalisation de l'invention, le tensioactif amphiphile comprend au moins un ester de sucrose choisi parmi le cocoate de sucrose, le monooctanoate de sucrose, le monodecanoate de sucrose, le monolaurate de sucrose, le monomyristate de sucrose, le monopalmitate de sucrose, le monostéarate de sucrose, le monooléate de sucrose, le monolinoléate de sucrose, le dioléate de sucrose, le dipalmitate de sucrose, le distéarate de sucrose, le dilaurate de sucrose, le dilinoléate de sucrose, le tristéarate de sucrose, le palmitostéarate de sucrose et leurs mélanges.

Selon un autre mode particulier de réalisation de l'invention, le tensioactif amphiphile comprend au moins un éther de glucose choisi parmi les alkyl-polyglucosides.

Comme exemples de tensioactifs amphiphiles, on peut utiliser en particulier un mélange de cocoate de sucrose et de stéarate de sorbitan, et en particulier le mélange de cocoate de sucrose et de stéarate de sorbitan commercialisé sous la dénomination Arlatone 2121 par la société ICI. Comme exemples de tensioactifs amphiphiles, on peut citer aussi le cocoate de sucrose vendu par la société Croda sous la dénomination Crodesta SL40 ; les stéarates de sucrose vendus par la société Croda sous les dénominations Crodesta F160, F140, F110, F90 et F70, désignant respectivement les palmitostéarates de sucrose formés de 73 % monoester et 27 % di-,triester, de 61 % monoester et 39 % di-tri-tétraester, de 52 % monoester et 48 % di-tri-tétraester, de 45 % monoester et de 55 % di-tri-tétraester, de 39 % monoester et de 61 % de di-, tritétraester ; le monolaurate de sucrose ; le monostéarate de sucrose ; le distéarate de sucrose ; le tristéarate de sucrose et leurs mélanges, tels que les produits commercialisés par la société Croda sous les dénominations Crodesta F50, F70, F110, F160 ayant respectivement un HLB (Hydrophilic Lipophilic Balance) de 5, 7, 11 et 16 ; le béhénate de sucrose vendu par la société MITSUBISHI sous la référence Ryoto Sugar ester B-370, formé de 20 % monoester et 80 % di-tri- etpolyester ; le distéarate de méthyl glucose et de polyglycérol-3, vendu par la société Goldschmidt sous la dénomination de Tegocare 450 ; l'o-hexadécanoyle-6-D-glucoside de méthyle ; l'o-hexadécanoyle-6-D-maltoside ; le « Sucrose polycottonseedate » (nom CTFA ; et leurs mélanges.

Le tensioactif amphiphile peut être constitué d'un mélange de ces différents tensioactifs préférés.

La phase aqueuse des compositions selon l'invention comprend au moins de l'eau. La quantité de phase aqueuse va généralement de 40 à 99 % en poids par rapport au poids total de la composition et de préférence de 60 à 95 % en poids par rapport au poids total de la composition. La quantité d'eau peut représenter tout ou une partie de la phase aqueuse et elle est généralement d'au moins 30 % en poids par rapport au poids total de la composition.

Les compositions de l'invention peuvent contenir dans la phase aqueuse ou dans la phase huileuse, un ou plusieurs solvants organiques, hydrophiles, lipophiles et/ou amphiphiles, physiologiquement acceptables, c'est-à-dire bien tolérés et donnant un toucher cosmétiquement acceptable.

Les solvants organiques peuvent représenter de 1 à 50 % et de préférence de 2 à 20 % du poids total de la composition. Les solvants organiques peuvent être choisis dans le groupe constitué par les solvants organiques hydrophiles, les solvants organiques lipophiles, les solvants amphiphiles, ou leurs mélanges.

Parmi les solvants organiques, on peut citer par exemple les mono-alcools inférieurs linéaires ou ramifiés ayant de 1 à 8 atomes de carbone, comme l'éthanol, le propanol, le butanol, l'isopropanol, l'isobutanol ; les polyols tels que le propylène glycol, l'isoprène glycol, le butylène glycol, le glycérol, le sorbitol ; les mono- ou di-alkyle isosorbide dont les groupements alkyle ont de 1 à 5 atomes de carbone comme le diméthyl isosorbide ; les polyéthylène glycols notamment ceux ayant de 6 à 80 oxydes d'éthylène ; les éthers d'éthylène glycol comme le diéthylène glycol mono-méthyl ou mono-éthyl éther ; les éthers de propylène glycol comme le dipropylène glycol méthyl éther ; les esters et éthers de polyol, tels que les esters de polypropylène glycol (PPG) et plus spécialement les esters de polypropylène glycol (PPG) et d'acide gras, les éthers de PPG et d'alcool gras comme le PPG-23 oléyl éther et le PPG-36 oléate ; les esters d'acide gras et d'alcool, tels que l'adipate de diisopropyle, l'adipate de dioctyle ; les benzoates d'alkyle ; et leurs mélanges.

La phase huileuse de la composition selon l'invention représente généralement de 3 à 60 % et de préférence de 5 à 30 % en poids par rapport au poids total de la composition.

La nature de la phase huileuse de l'émulsion selon l'invention n'est pas critique. La phase huileuse peut ainsi être constituée par tous les corps gras et notamment les huiles, classiquement utilisés dans les domaines cosmétique ou dermatologique. La phase huileuse comprend généralement au moins une huile.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R^{a}COOR^{b} et R^{a}OR^{b} dans laquelle R^{a} représente le reste d'un acide gras comportant de 8 à 29 atomes de carbone, et R^{b} représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèheglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12, le ceteareth-12 et le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane; les perfluoroalcanes tels que le dodécafluoropentane et le tétradécafluorohexane, vendus sous les dénominations de "PF 5050®" et "PF 5060®" par la Société 3M, ou encore le bromoperfluorooctane vendu sous la dénomination "FORALKYL®" par la Société Atochem ; le nonafluorométhoxybutane vendu sous la dénomination "MSX 4518®" par la Société 3M et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition de l'invention contient au moins une huile de silicone, de préférence une huile de silicone volatile qui peut être choisie par exemple parmi les polydiméthylsiloxanes cycliques ou linéaires, et leurs mélanges. Les polydiméthylsiloxanes cycliques ou cyclométhicones comportent d'environ 3 à 9 atomes de silicium, et de préférence de 4 à 6 atomes de silicium et peuvent être par exemple le cyclohexadiméthyl-siloxane et le cyclopentadiméthylsiloxane. Les polydiméthylsiloxanes linéaires volatiles comportent de préférence d'environ 3 à 9 atomes de silicium. Les polydiméthylsiloxanes linéaires volatiles ont généralement une viscosité à 25°C inférieure ou égale à 5 cSt tandis que les cyclométhicones ont généralement une viscosité à 25°C inférieure ou égale à 10 cSt.

De façon connue, toutes les compositions de l'invention peuvent contenir un ou plusieurs des adjuvants habituels dans les domaines cosmétique et dermatologique, tels que des agents gélifiants et/ou épaississants hydrophiles ou lipophiles ; des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des pigments ; des agents filmogènes ; des matières colorantes, et leurs mélanges. Les quantités de ces différents adjuvants sont celles classiquement utilisées dans les domaines considérés.

Comme gélifiants, on peut citer par exemple les polymères hydrophiles tels que les polymères carboxyvinyliques comme les carbomers ; les polysaccharides comme les gommes de guar, de xanthane et les dérivés de cellulose comme par exemple l'hydroxyéthylcellulose ; les dérivés siliconés hydrosolubles ou hydrodispersibles comme les silicones acryliques et les silicones cationiques. On peut aussi utiliser des gélifiants lipophiles, tels que les argiles modifiés ou les polysaccharides modifiés.

Comme actifs utilisables dans la composition de l'invention, on peut citer par exemple les agents hydratants tels que les hydrolysats de protéines et les polyols comme la glycérine, les glycols comme les polyéthylène glycols, et les dérivés de sucre ; les extraits naturels ; les anti-inflammatoires ; les oligomères procyannidoliques ; les vitamines comme la vitamine A (rétinol), la vitamine E (tocophérol), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; les filtres solaires ; l'hydrocortisone ; la mélatonine ; les extraits d'algues, de champignons, de végétaux, de levures, de bactéries ; les enzymes ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants comme les fibres ; les agents tenseurs ; et leurs mélanges.

Comme exemples de stéroïdes, on peut citer la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Les filtres solaires (ou filtres U.V.) peuvent être choisis parmi les filtres organiques, les filtres physiques et leurs mélanges.

Comme filtres solaires chimiques utilisables dans la composition de l'invention, la composition de l'invention peut comprendre tous les filtres UVA et UVB utilisables dans le domaine cosmétique.

Comme filtres UVB, on peut citer par exemple :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, commercialisé par la société Givaudan sous la dénomination Parsol MCX;
(3) les dérivés de β,β-diphénylacrylate liquides, en particulier l'α-cyano-β,β diphénylacrylate de 2-éthylhexyle ou octocrylène, commercialisé par la société BASF sous la dénomination UVINUL N539 ;
(4) les dérivés de l'acide p-aminobenzoïque ;
(5) le 4-méthyl benzylidène camphre commercialisé par la société Merck sous la dénomination EUSOLEX 6300 ;
(6) l'acide 2-phénylbenzimidazole 5-sulfonique commercialisé sous la dénomination EUSOLEX 232 par la société Merck ;
(7) les dérivés de 1,3,5-triazine, en particulier :
   - la 2,4,6-tris[p-(2'-éthylhexyl-1'-oxycarbonyl)anilino]-1,3,5-triazine commercialisé par la société BASF sous la dénomination UVINUL T150, et
   - le dioctyl butamido triazone commercialisé par la société Sigma 3V sous la dénomination UVASORB HEB ;
(8) les mélanges de ces filtres.

Comme filtres UVA, on peut citer par exemple :
(1) les dérivés de dibenzoylméthane, en particulier le 4-(ter.-butyl) 4'-méthoxy dibenzoylméthane commercialisé par la société Givaudan sous la dénomination PARSOL 1789 ;
(2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
(3) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), commercialisé sous la dénomination UVINUL M40 par la société BASF ;
   - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12).
(4) les dérivés silanes ou les polyorganosiloxanes à groupement benzophénone ;
(5) les anthranilates, en particulier l'anthranilate de menthyle commercialisé par la société Haarman & Reiner sous la dénomination NEO HELIOPAN MA ;
(6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer ;
(7) les dérivés siliciés de benzimidazolyl-benzazoles N-substitués ou de benzofuranyl-benzazoles, et en particulier :
   - le 2-[1 -[3-[1,3,3,3-tétraméthyl-1-[(triméthylsilyl)oxy]disiloxanyl]propyl]-1 H-benzimidazol-2-yl]-benzoxazole ;
   - le 2-[1-[3-[1,3,3,3-tétraméthyl-1 -[(triméthylsilyl)oxy]disiloxanyl]propyl]-1 H-benzimidazol-2-yl]-benzothiazole ;
   - le 2-[1-(3-trimethylsilanyl-propyl)-1H-benzimidazol-2-yl]-benzoxazole ;
   - le 6-méthoxy-1,1'-bis-(3-triméthylsilanyl-propyl)-1 H,l'H-[2,2']bibenzimidazolyl-benzoxazole ;
   - le 2-[1-(3-trimethylsilanyl-propyl)-1H-benzimidazol-2-yl]-benzothiazole ; qui sont décrits dans la demande de brevet EP-A-1 028 120 ;
(8) les dérivés de triazine, et en particulier la 2,4-bis [4-(2-éthyl-hexyloxy)-2-hydroxy]-phényl]-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
(9) leurs mélanges.

On peut aussi utiliser un mélange de plusieurs de ces filtres et un mélange de filtres UVB et de filtres UVA et aussi des mélanges avec des filtres physiques.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm. On utilise de préférence dans la composition de l'invention des nano-pigments.

Ces pigments sont par ailleurs notamment utilisés dans les compositions de maquillage, éventuellement après avoir été traités de manière à rendre leur surface hydrophobe; ce traitement peut être effectué selon les méthodes connues de l'homme du métier ; les pigments peuvent notamment être enrobés par des composés siliconés tels que des PDMS et/ou par des polymères.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gels, de lotions, de laits, de crèmes plus ou moins onctueuses, de pâtes. Ces compositions sont préparées selon les méthodes usuelles.

La composition présente de préférence un pH qui respecte la peau et qui va généralement de 3 à 8 et de préférence de 4,5 à 7.

Les compositions de l'invention peuvent être utilisées comme produit de soin, de traitement, de protection, de nettoyage, de démaquillage, et /ou de maquillage des matières kératiniques (peau, cheveux, cuir chevelu, cils, sourcils, ongles ou muqueuses) tels que des crèmes de protection, de traitement ou de soin pour le visage, pour les mains ou pour le corps, des laits corporels de protection ou de soin, des gels ou mousses pour le soin de la peau et/ou des muqueuses (lèvres).

Les compositions de l'invention peuvent contenir des filtres solaires et être ainsi également utilisées comme produit de protection solaire.

Les compositions peuvent être utilisées comme produits pour le maquillage, notamment le maquillage de la peau, des sourcils, des cils et des lèvres, tels que des crèmes pour le visage, des fonds de teint, des mascaras, des rouges à lèvres. De tels produits contiennent généralement des pigments.

Les compositions selon l'invention peuvent être également utilisées comme produits rincés ou comme produits non-rincés pour le nettoyage de la peau du visage et/ou du corps et/ou pour le nettoyage des cheveux, par exemple comme produits capillaires y compris pour le soin et le conditionnement des cheveux.

L'invention a pour objet l'utilisation cosmétique d'une composition cosmétique telle que définie ci-dessus, comme produit capillaire rincé ou non-rincé.

L'invention a pour objet l'utilisation cosmétique d'une composition cosmétique telle que définie ci-dessus, comme produit de nettoyage et/ou de démaquillage de la peau et/ou des yeux.

L'invention a aussi pour objet l'utilisation cosmétique d'une composition cosmétique telle que définie ci-dessus, comme produit de soin pour la peau, les cheveux, le cuir chevelu, des cils, des sourcils, des ongles ou des muqueuses.

L'invention a aussi pour objet l'utilisation cosmétique d'une composition cosmétique telle que définie ci-dessus, comme produit de maquillage.

L'invention a aussi pour objet l'utilisation cosmétique d'une composition cosmétique telle que définie ci-dessus, comme produit de protection solaire (protection contre le soleil et/ou les U.V. des appareils à bronzer).

Un autre objet de l'invention est un procédé de traitement cosmétique (non-thérapeutique) d'une matière kératinique (peau, cuir chevelu, cheveux, cils, sourcils, ongles ou muqueuses), caractérisé en ce qu'on applique sur la matière kératinique, une composition cosmétique telle que définie ci-dessus. La matière kératinique est notamment la peau.

Les exemples qui suivent permettront de mieux comprendre l'invention, sans toutefois présenter un caractère limitatif. Les quantités indiquées sont en % en poids, sauf mention contraire.

### Exemple 1 : Crème

| *Phase A (phase huileuse)* | |
|---|---|
| Mélange de cocoyl polyglucoside et d'alcool cetylique, stéarylique (35/65) (Montanov 82 de la société SEPPIC) | 3 % |
| Huile de soja | 10 % |
| Cyclohexadiméthylsiloxane | 5 % |
| Huile de vaseline | 10 % |
| Acide stéarique | 0,5 % |

| *Phase B (phase aqueuse)* | |
|---|---|
| Glycérine | 5 % |
| Carbomer | 0,5 % |
| Triéthanolamine | 0,5 % |
| Eau | qsp 100 % |

| *Phase C* | |
|---|---|
| HMW2220 (Dow Corning) | 2 % |
| Parfum | 0,1 % |

Mode opératoire : On prépare la phase B à chaud sous agitation. On y ajoute la phase A. On refroidit puis on ajoute sous faible cisaillement de la dispersion, la phase C.

On obtient une crème blanche, très douce à l'application sans toucher gras, donnant une bonne sensation d'hydratation après application.

### Exemple 2 : Crème

| *Phase A (phase huileuse)* | |
|---|---|
| Mono-di-palmito stearate de sucrose (Tegosoft PSE 141 G de Goldschmidt) | 2 % |
| Alcool stéarylique | 1 % |
| Cyclohexadiméthylsiloxane | 2 % |
| Isohexadécane | 5 % |
| Acide stéarique | 0,5 % |

| *Phase B (phase aqueuse)* | |
|---|---|
| Glycérine | 5 % |
| Carbomer | 0,5 % |
| Triéthanolamine | 0,5 % |
| Eau | qsp 100 % |

| *Phase C* | |
|---|---|
| HMW2220 (Dow Corning) | 4 % |
| Parfum | 0,1 % |

Mode opératoire : On prépare la phase B à chaud sous agitation. On y ajoute la phase A. On refroidit puis on ajoute sous faible cisaillement de la dispersion, la phase C.

On obtient une crème blanche, très douce à l'application sans toucher gras, donnant une bonne sensation d'hydratation après application.

### Exemple 3 : Fluide pulvérisable

| *Phase A (phase huileuse)* | |
|---|---|
| Silicone volatile | 10 % |
| Huile minérale | 10 % |
| Lécthine | 3 % |
| Lécithine hydrogénée | 1 % |

| *Phase B (phase aqueuse)* | |
|---|---|
| Glycérine | 5 % |
| Eau | qsp 100 % |
| Conservateur | qs |

| *Phase C* | |
|---|---|
| HMW2220 (Dow Corning) | 2 % |
| Parfum | 0,1 % |

Mode opératoire : On solubilise les lécithines dans la phase grasse à chaud (70°C) sous agitation. Par ailleurs, on prépare la phase aqueuse à chaud. On verse la phase A dans la phase B et on fait une pré-émulsion sous agitation. On passe la pré-émulsion dans un appareil haute pression (500 bars, 2 passages). On refroidit, puis on ajoute la phase C sous faible agitation.

On obtient un fluide, très frais et léger à l'application, laissant la peau douce et matte avec une bonne sensation d'hydratation. Ce fluide peut être appliqué directement ou par pulvérisation.

## Revendications

1. Composition sous forme d'émulsion huile-dans-eau comprenant dans un milieu physiologiquement acceptable, une phase huileuse dispersée dans une phase aqueuse, **caractérisée par le fait que** la phase aqueuse comprend des particules de copolymère siliconé non réticulé et au moins un tensioactif amphiphile susceptible de former des cristaux liquides, **par le fait que** la phase huileuse contient au moins une huile, et **par le fait que** le copolymère siliconé est obtenu par réaction d'extension de chaîne, en présence d'un catalyseur, à partir d'au moins :
- (a) un polysiloxane (i) qui est le diméthylvinyl-siloxy-polydiméthylsiloxane; et
- (b) un composé organosiliconé (ii) qui réagit avec le polysiloxane (i) par réaction d'extension de chaîne, et qui est un organohydrogenopolysiloxane liquide de formule (II) : 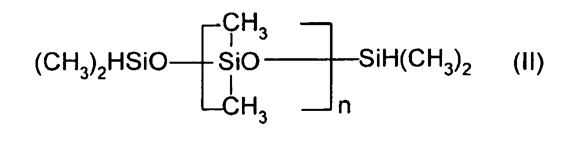
où n est un nombre entier supérieur à 1 et de préférence supérieur à 10.

2. Composition selon la revendication 1, **caractérisée par le fait que** la quantité de particules de copolymère siliconé va de 0,01 à 15 % en poids par rapport au poids total de la composition.

3. Composition selon la revendication 1 ou 2, **caractérisée par le fait que** les particules de copolymère siliconé présentent une taille moyenne en nombre inférieure ou égale à 2 microns.

4. Composition selon l'une quelconque des revendications précédentes, **caractérisée par le fait que** les particules de copolymère siliconé se présentent sous forme d'une dispersion aqueuse obtenue par mélange d'eau, d'au moins un émulsifiant, du polysiloxane (i), du composé organosiliconé (ii) et d'un catalyseur.

5. Composition selon la revendication précédente, **caractérisée par le fait que** l'émulsifiant est un émulsifiant non ionique choisi parmi les polyalkylène glycols éthers d'alcool gras, comportant de 8 à 30 atomes de carbone ; les alkylesters de sorbitan polyoxyalkylénés, où le radical alkyle comporte de 8 à 30 atomes de carbone; les alkylesters polyoxyalkylénés, où le radical alkyle comporte de 8 à 30 atomes de carbone ; les polyéthylène glycols ; les polypropylène glycols ; les diéthylène glycols ; et leurs mélanges.

6. Composition selon la revendication précédente, **caractérisée par le fait que** l'émulsifiant est choisi parmi les polyéthylène glycols éthers d'alcools comportant 12 ou 13 atomes de carbone et de 3 à 50 motifs oxyéthylénés, et leurs mélanges.

7. Composition selon l'une quelconque des revendications précédentes, **caractérisée par le fait que** la quantité de tensioactif(s) amphiphile(s) va de 0,05 à 20 % en poids de matière active par rapport au poids total de la composition.

8. Composition selon l'une quelconque des revendications précédentes, **caractérisée par le fait que** le tensioactif amphiphile est choisi parmi les tensioactifs anioniques, cationiques, amphotères, non ioniques et leurs mélanges.

9. Composition selon l'une quelconque des revendications précédentes, **caractérisée par le fait que** le tensioactif amphiphile comporte une partie hydrophile, et une partie lipophile comprenant une chaîne saturée ou insaturée, linéaire ou ramifiée, ayant de 8 à 30 atomes de carbone.

10. Composition selon la revendication précédente, **caractérisée par le fait que** la partie hydrophile du tensioactif amphiphile comporte un groupe choisi parmi les chaînes oxyéthylénées, les groupes polyglycérolés, les groupes polyols, les groupes polyols oxalkylénés, et leurs mélanges.

11. Composition selon l'une quelconque des revendications précédentes, **caractérisée par le fait que** le tensioactif amphiphile est un tensioactif non ionique choisi parmi les alkyléthers de polyglycérol ; les alkyl phénols éthoxylés ; les esters de polyols et leurs dérivés oxyalkylénés ; les éthers d'alcools gras et de sucre ; les silicones oxyéthylénés ; les polymères blocs d'oxyde d'éthylène et d'oxyde de propylène ; et leurs mélanges.

12. Composition selon l'une quelconque des revendications précédentes, **caractérisée par le fait que** le tensioactif amphiphile est un tensioactif anionique choisi parmi les acides et sels d'acides carboxyliques comportant de 8 à 22 atomes de carbone ; les esters et sels d'acide phosphonique ou d'acide phosphorique ; les diesters d'acide phosphorique ; les alkane sulfonates et leurs sels ; les sulfosuccinates ; les alkyl sulfates et leurs sels ; les derivés de la phosphotidylcholine, et leurs mélanges.

13. Composition selon l'une quelconque des revendications précédentes, **caractérisée par le fait que** le tensioactif amphiphile est un tensioactif cationique choisi parmi les dérivés quaternaires d'alkyl-imidazolines ; les amines éthoxylées ; les alkylamines ; les dérivés quaternaires d'alkylbenzyle ; et leurs mélanges.

14. Composition selon l'une quelconque des revendications précédentes, **caractérisée par le fait que** le tensioactif amphiphile est un tensioactif amphotère ou zwitterionique choisi parmi les alkyl betaïnes, les alkylamido propyl bétaines ; et leurs mélanges.

15. Composition selon l'une quelconque des revendications précédentes, **caractérisée par le fait que** le tensioactif amphiphile est choisi parmi les esters de sucre, les éthers de sucre, les esters de sorbitan et leurs mélanges.

16. Composition selon l'une quelconque des revendications précédentes, **caractérisée par le fait que** le tensioactif amphiphile comprend au moins un ester de sucrose choisi parmi le cocoate de sucrose, le monooctanoate de sucrose, le monodecanoate de sucrose, le monolaurate de sucrose, le monomyristate de sucrose, le monopalmitate de sucrose, le monostéarate de sucrose, le monooléate de sucrose, le monolinoléate de sucrose, le dioléate de sucrose, le dipalmitate de sucrose, le distéarate de sucrose, le dilaurate de sucrose, le dilinoléate de sucrose, le tristéarate de sucrose, le palmitostéarate de sucrose, et leurs mélanges.

17. Composition selon l'une quelconque des revendications précédentes, **caractérisée par le fait que** le tensioactif amphiphile est un mélange de stéarate de sorbitan et de cocoate de sucrose.

18. Composition selon l'une quelconque des revendications précédentes, **caractérisée par le fait que** le tensioactif amphiphile comprend au moins un éther de glucose choisi parmi les alkyl-polyglucosides.

19. Composition selon l'une quelconque des revendications précédentes, **caractérisée par le fait que** la phase aqueuse représente de 40 à 99 % et de préférence de 60 à 95 % en poids par rapport au poids total de la composition.

20. Composition selon l'une quelconque des revendications précédentes, **caractérisée par le fait qu'**elle comprend un ou plusieurs solvants organiques.

21. Composition selon la revendication précédente, **caractérisée par le fait que** le solvant est choisi parmi les mono-alcools inférieurs linéaires ou ramifiés ayant de 1 à 8 atomes de carbone ; les polyols ; les mono- ou di-alkyle isosorbide dont les groupements alkyle ont de 1 à 5 atomes de carbone ; les polyéthylène glycols ayant de 6 à 80 oxydes d'éthylène ; les éthers d'éthylène glycol ; les éthers de propylène glycol ; les esters et éthers de polyol ; les esters d'acide gras et d'alcool ; et leurs mélanges.

22. Composition selon la revendication 20 ou 21, **caractérisée par le fait que** la quantité de solvant(s) va de 1 à 50 % en poids par rapport au poids total de la composition.

23. Composition selon l'une quelconque des revendications précédentes, **caractérisée par le fait que** la phase huileuse représente de 3 à 60 % et de préférence de 5 à 30 % en poids par rapport au poids total de la composition.

24. Composition selon la revendication précédente, **caractérisée par le fait que** la phase huileuse comprend au moins une huile de silicone volatile.

25. Composition selon l'une quelconque des revendications précédentes, **caractérisée par le fait qu'**elle comprend en outre un ou plusieurs adjuvants choisis parmi les agents gélifiants ; les agents hydratants ; les émollients ; les actifs ; les agents anti-radicaux libres ; les séquestrants ; les antioxydants ; les conservateurs ; les agents alcanisants ou acidifiants ; les parfums ; les pigments ; les agents filmogènes ; les matières colorantes, et leurs mélanges.

26. Composition selon l'une quelconque des revendications précédentes, **caractérisée par le fait qu'**elle constitue une composition cosmétique ou dermatologique.

27. Composition selon l'une quelconque des revendications précédentes, **caractérisée par le fait qu'**elle constitue un produit de soin, de traitement, de protection, de nettoyage, de démaquillage et /ou de maquillage des matières kératiniques.

28. Composition selon la revendication précédente, **caractérisée par le fait que** la matière kératinique est la peau.

29. Utilisation cosmétique d'une composition selon l'une quelconque des revendications 1 à 25, comme produit de soin pour la peau, les cheveux, le cuir chevelu, des cils, des sourcils, des ongles ou des muqueuses.

30. Utilisation cosmétique d'une composition selon l'une quelconque des revendications 1 à 25, comme produit de maquillage.

31. Utilisation cosmétique d'une composition selon l'une quelconque des revendications 1 à 25, comme produit de protection solaire.

32. Utilisation cosmétique d'une composition selon l'une quelconque des revendications 1 à 25, comme produit capillaire rincé ou non-rincé.

33. Utilisation cosmétique d'une composition selon l'une quelconque des revendications 1 à 25, comme produit de nettoyage et/ou de démaquillage de la peau et/ou des yeux.

34. Procédé de traitement cosmétique d'une matière kératinique, **caractérisé par le fait qu'**on applique sur la matière kératinique, une composition telle que définie selon l'une quelconque des revendications 1 à 25.

35. Procédé selon la revendication précédente, **caractérisé par le fait que** la matière kératinique est la peau.

## Claims

1. Composition in the form of an oil-in-water emulsion comprising, in a physiologically acceptable medium, an oily phase dispersed in an aqueous phase, **characterized in that** the aqueous phase comprises particles of uncrosslinked silicone copolymer and at least one amphiphilic surfactant capable of forming liquid crystals, **in that** the oily phase comprises at least one oil, and **in that** the silicone copolymer is obtained by a chain-extension reaction in the presence of a catalyst starting from at least:
- (a) one polysiloxane (i) which is dimethylvinylsiloxypolydimethylsiloxane; and
- (b) one organosilicone compound (ii) which reacts with the polysiloxane (i) by a chain-extension reaction and which is a liquid organohydropolysiloxane of formula (II) : 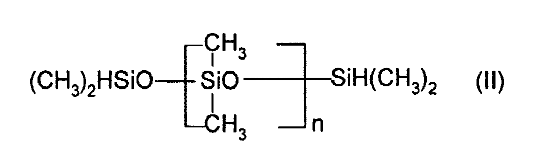
where n is an integer greater than 1 and preferably greater than 10.

2. Composition according to Claim 1, **characterized in that** the amount of silicone copolymer particles ranges from 0.01 to 15% by weight with respect to the total weight of the composition.

3. Composition according to Claim 1 or 2,
**characterized in that** the silicone copolymer particles exhibit a number-average size of less than or equal to 2 microns.

4. Composition according to any one of the preceding claims, **characterized in that** the silicone copolymer particles are provided in the form of an aqueous dispersion obtained by mixing water, at least one emulsifier, the polysiloxane (i), the organosilicone compound (ii) and a catalyst.

5. Composition according to the preceding claim, **characterized in that** the emulsifier is a nonionic emulsifier chosen from polyalkylene glycol ethers of a fatty alcohol comprising from 8 to 30 carbon atoms; polyoxyalkylenated alkyl esters of sorbitan, where the alkyl radical comprises from 8 to 30 carbon atoms; polyoxyalkylenated alkyl esters, where the alkyl radical comprises from 8 to 30 carbon atoms; polyethylene glycols; polypropylene glycols; diethylene glycols; and their mixtures.

6. Composition according to the preceding claim, **characterized in that** the emulsifier is chosen from polyethylene glycol ethers of alcohols comprising 12 or 13 carbon atoms and of 3 to 50 oxyethylene units, and their mixtures.

7. Composition according to any one of the preceding claims, **characterized in that** the amount of amphiphilic surfactant(s) ranges from 0.05 to 20% by weight of active material with respect to the total weight of the composition.

8. Composition according to any one of the preceding claims, **characterized in that** the amphiphilic surfactant is chosen from anionic, cationic, amphoteric or nonionic surfactants and their mixtures.

9. Composition according to any one of the preceding claims, **characterized in that** the amphiphilic surfactant comprises a hydrophilic part and a lipophilic part comprising a saturated or unsaturated and linear or branched chain having from 8 to 30 carbon atoms.

10. Composition according to the preceding claim, **characterized in that** the hydrophilic part of the amphiphilic surfactant comprises a group chosen from oxyethylene chains, polyglycerol groups, polyol groups, oxyalkylenated polyol groups, and their mixtures.

11. Composition according to any one of the preceding claims, **characterized in that** the amphiphilic surfactant is a nonionic surfactant chosen from polyglycerol alkyl ethers; ethoxylated alkylphenols; polyol esters and their oxyalkylenated derivatives; ethers of fatty alcohols and of a sugar; oxyethylenated silicones; block polymers of ethylene oxide and of propylene oxide; and their mixtures.

12. Composition according to any one of the preceding claims, **characterized in that** the amphiphilic surfactant is an anionic surfactant chosen from carboxylic acids and salts of carboxylic acids comprising from 8 to 22 carbon atoms; esters and salts of phosphonic acid or of phosphoric acid; phosphoric acid diesters; alkanesulphonates and their salts; sulphosuccinates; alkyl sulphates and their salts; phosphatidylcholine derivatives; and their mixtures.

13. Composition according to any one of the preceding claims, **characterized in that** the amphiphilic surfactant is a cationic surfactant chosen from quaternary alkylimidazoline derivatives; ethoxylated amines; alkylamines; quaternary alkylbenzyl derivatives; and their mixtures.

14. Composition according to any one of the preceding claims, **characterized in that** the amphiphilic surfactant is an amphoteric or zwitterionic surfactant chosen from alkyl betaines, alkylamidopropyl betaines; and their mixtures.

15. Composition according to any one of the preceding claims, **characterized in that** the amphiphilic surfactant is chosen from sugar esters, sugar ethers, sorbitan esters and their mixtures.

16. Composition according to any one of the preceding claims, **characterized in that** the amphiphilic surfactant comprises at least one sucrose ester chosen from sucrose cocoate, sucrose monooctanoate, sucrose monodecanoate, sucrose monolaurate, sucrose monomyristate, sucrose monopalmitate, sucrose monostearate, sucrose monooleate, sucrose monolinoleate, sucrose dioleate, sucrose dipalmitate, sucrose distearate, sucrose dilaurate, sucrose dilinoleate, sucrose tristearate, sucrose palmitate/stearate, and their mixtures.

17. Composition according to any one of the preceding claims, **characterized in that** the amphiphilic surfactant is a mixture of sorbitan stearate and of sucrose cocoate.

18. Composition according to any one of the preceding claims, **characterized in that** the amphiphilic surfactant comprises at least one glucose ether chosen from alkylpolyglucosides.

19. Composition according to any one of the preceding claims, **characterized in that** the aqueous phase represents from 40 to 99% and preferably from 60 to 95% by weight with respect to the total weight of the composition.

20. Composition according to any one of the preceding claims, **characterized in that** it comprises one or more organic solvents.

21. Composition according to the preceding claim, **characterized in that** the solvent is chosen from linear or branched lower monoalcohols having from 1 to 8 carbon atoms; polyols; mono- or dialkyl isosorbide, the alkyl groups of which have from 1 to 5 carbon atoms; polyethylene glycols having from 6 to 80 ethylene oxides; ethylene glycol ethers; propylene glycol ethers; polyol esters and ethers; alkyl fatty acid esters; and their mixtures.

22. Composition according to Claim 20 or 21,
**characterized in that** the amount of solvent(s) ranges from 1 to 50% by weight with respect to the total weight of the composition.

23. Composition according to any one of the preceding claims, **characterized in that** the oily phase represents from 3 to 60% and preferably from 5 to 30% by weight with respect to the total weight of the composition.

24. Composition according to the preceding claim, **characterized in that** the oily phase comprises at least one volatile silicone oil.

25. Composition according to any one of the preceding claims, **characterized in that** it additionally comprises one or more adjuvants chosen from gelling agents; moisturizing agents; emollients; active principles; agents for combating free radicals; sequestering agents; antioxidants; preservatives; basifying or acidifying agents; fragrances; pigments; film-forming agents; colouring materials; and their mixtures.

26. Composition according to any one of the preceding claims, **characterized in that** it constitutes a cosmetic or dermatological composition.

27. Composition according to any one of the preceding claims, **characterized in that** it constitutes a product for caring for, treating, protecting, cleaning, removing make-up from and/or making-up keratinous substances.

28. Composition according to the preceding claim, **characterized in that** the keratinous substance is the skin.

29. Cosmetic use of a composition according to any one of Claims 1 to 25 as product for caring for the skin, hair, scalp, eyelashes, eyebrows, nails or mucous membranes.

30. Cosmetic use of a composition according to any one of Claims 1 to 25 as make-up product.

31. Cosmetic use of a composition according to any one of Claims 1 to 25 as sun protection product.

32. Cosmetic use of a composition according to any one of Claims 1 to 25 as rinse-out or leave-in hair product.

33. Cosmetic use of a composition according to any one of Claims 1 to 25 as product for cleaning and/or removing make-up from the skin and/or eyes.

34. Process for the cosmetic treatment of a keratinous substance, **characterized in that** a composition as defined according to any one of Claims 1 to 25 is applied to the keratinous substance.

35. Process according to the preceding claim,
**characterized in that** the keratinous substance is the skin.

## Patentansprüche

1. Zusammensetzung in Form einer Öl-in-Wasser-Emulsion, die in einem physiologisch akzeptablen Medium eine in einer wässerigen Phase dispergierte Ölphase enthält,
**dadurch gekennzeichnet, dass** die wässerige Phase Partikel aus einem unvernetzten Siliconcopolymer und mindestens ein amphiphiles Tensid enthält, das Flüssigkristalle bilden kann, dass die Ölphase mindestens ein Öl enthält und dass das Siliconcopolymer durch Kettenverlängerungsreaktion in Gegenwart eines Katalysators erhalten ist aus mindestens
- (a) einem Polysiloxan (i), bei dem es sich um Dimethylvinylsiloxy-polydimethylsiloxan handelt, und
- (b) einer Organosiliconverbindung (ii), die mit dem Polysiloxan (i) durch Kettenverlängerungsreaktion reagiert und bei der es sich um ein flüssiges Organohydrogenpolysiloxan der Formel (ii) handelt, 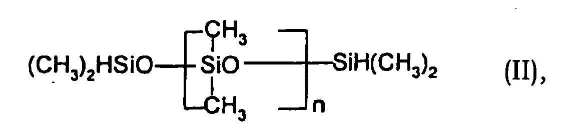 in der n eine ganze Zahl größer 1 und vorzugsweise größer als 10 bedeutet.

2. Zusammensetzung nach Anspruch 1, **dadurch gekennzeichnet, dass** die Menge an Partikeln aus dem Siliconcopolymer im Bereich von 0,01 bis 15 Gew.-% liegt, bezogen auf das Gesamtgewicht der Zusammensetzung.

3. Zusammensetzung nach Anspruch 1 oder 2, **dadurch gekennzeichnet, dass** die Partikel des Siliconcopolymers ein Zahlenmittel der Teilchengröße von 2 µm oder weniger aufweisen.

4. Zusammensetzung nach einem der vorhergehenden Ansprüche,
**dadurch gekennzeichnet, dass** die Partikel des Silicon copolymers in Form einer wässerigen Dispersion vorliegen, die durch Mischen von Wasser, mindestens eines Emulgators, des Polysiloxans (i), der Organosiliconverbindung (ii) und eines Katalysators erhalten ist.

5. Zusammensetzung nach dem vorhergehenden Anspruch,
**dadurch gekennzeichnet, dass** der Emulgator ein nichtionischer Emulgator ist, der ausgewählt ist unter Fettalkoholpolyalkylengycolethern mit 8 bis 30 Kohlenstoffatomen; polyoxyalkylenierten Sorbitan-alkylestern, bei denen die Alkylgruppe 8 bis 30 Kohlenstoffatomen aufweist; polyoxyalkylenierten Alkylestern, bei denen die Alkylgruppe 8 bis 30 Kohlenstoffatome aufweist; Polyethylenglycolen; Polypropylenglycolen und Diethylenglycolen sowie Gemischen dieser Verbindungen.

6. Zusammensetzung nach dem vorhergehenden Anspruch,
**dadurch gekennzeichnet, dass** der Emulgator unter Polyethylenglycolethern von Alkoholen mit 12 oder 13 Kohlenstoffatomen und 3 bis 50 Oxyethylen-Einheiten sowie ihren Gemischen ausgewählt ist.

7. Zusammensetzung nach einem der vorhergehenden Ansprüche,
**dadurch gekennzeichnet, dass** die Menge des amphiphilen Tensids oder der amphiphilen Tenside im Bereich von 0,05 bis 20 Gew.-% Wirkstoff liegt, bezogen auf das Gesamtgewicht der Zusammensetzung.

8. Zusammensetzung nach einem der vorhergehenden Ansprüche,
**dadurch gekennzeichnet, dass** das amphiphile Tensid unter anionischen, kationischen, amphoteren und nichtionischen Tensiden und ihren Gemischen ausgewählt ist.

9. Zusammensetzung nach einem der vorhergehenden Ansprüche,
**dadurch gekennzeichnet, dass** das amphiphile Tensid einen hydrophilen Teil und einen lipophilen Teil aufweist, d er eine geradkettige oder verzweigte, gesättigte oder ungesättigte Kette mit 8 bis 30 Kohlenstoffatomen aufweist.

10. Zusammensetzung nach dem vorhergehenden Anspruch,
**dadurch gekennzeichnet, dass** der hydrophile Teil des amphiphilen Tensids eine Gruppe aufweist, die ausgewählt ist unter oxyethylenierten Ketten, polyglycerylierten Gruppen, Polyolgruppen und oxyalkylenierten Polyolgruppen sowie Gemischen davon.

11. Zusammensetzung nach einem der vorhergehenden Ansprüche,
**dadurch gekennzeichnet, dass** das amphiphile Tensid ein nichtionisches Tensid ist, das ausgewählt ist unter Polyglycerinalkylethern; ethoxylierten Alkylphenolen; . Polyolestern und ihren oxyalkylenierten Derivaten; Ethern von Fettalkoholen mit Zuckern; oxyethylenierten Siliconen; Blockpolymeren von Ethylenoxid und Propylenoxid sowie Gemischen dieser Verbindungen.

12. Zusammensetzung nach einem der vorhergehenden Ansprüche,
**dadurch gekennzeichnet, dass** das amphiphile Tensid ein anionisches Tensid ist, das ausgewählt ist unter Carbonsäuren und ihren Salzen mit 8 bis 22 Kohlenstoffatomen; Estern und Salzen von Phosphonsäure oder Phosphorsäure; Phosphorsäurediestern; Alkansulfonaten und ihren Salzen; Sulfosuccinaten; Alkylsulfaten und ihren Salzen; Derivaten von Phosphatidylcholin sowie Gemischen dieser Verbindungen.

13. Zusammensetzung nach einem der vorhergehenden Ansprüche,
**dadurch gekennzeichnet, dass** das amphiphile Tensid ein kationisches Tensid ist, das ausgewählt ist unter quaternären Derivaten von Alkylimidazolinen; ethoxylierten Aminen; Alkylaminen; quaternären Derivaten von Alkylbenzol sowie Gemischen dieser Verbindungen.

14. Zusammensetzung nach einem der vorhergehenden Ansprüche,
**dadurch gekennzeichnet, dass** das amphiphile Tensid ein amphoteres oder zwitterionisches Tensid ist, das au sgewählt ist unter Alkylbetainen, Alkylamidopropylbetainen und Gemischen dieser Verbindungen.

15. Zusammensetzung nach einem der vorhergehenden Ansprüche,
**dadurch gekennzeichnet, dass** das amphiphile Tensid unter Zuckerestern, Zuckerethern, Sorbitanestern sowie Gemischen dieser Verbindungen ausgewählt ist.

16. Zusammensetzung nach einem der vorhergehenden Ansprüche,
**dadurch gekennzeichnet, dass** das amphiphile Tensid mindestens einen Sucroseester umfasst, der ausgewählt ist unter Sucrosecocoat, Sucrosemonooctanoat, Sucrosemonodecanoat, Sucrosemonolaurat, Sucrosemonomyristat, Sucrosemonopalmitat, Sucrosemonostearat, Sucrosemonooleat, Sucrosemonolinoleat, Sucrosedioleat, Sucrosedipalmitat, Sucrosedistearat, Sucrosedilaurat, Sucrosedilinoleat, Sucrosetristearat und Sucrosepalmitostearat sowie Gemischen dieser Verbindungen.

17. Zusammensetzung nach einem der vorhergehenden Ansprüche,
**dadurch gekennzeichnet, dass** das amphiphile Tensid ein Gemisch von Sorbitanstearat und Sucrosecocoat ist.

18. Zusammensetzung nach einem der vorhergehenden Ansprüche,
**dadurch gekennzeichnet, dass** das amphiphile Tensid mindestens einen Glucoseether umfasst, der unter den Alkylpolyglucosiden ausgewählt ist.

19. Zusammensetzung nach einem der vorhergehenden Ansprüche,
**dadurch gekennzeichnet, dass** die wässerige Phase 40 bis 99 Gew.-% und vorzugsweise 60 bis 95 Gew.-% ausmacht, bezogen auf das Gesamtgewicht der Zusammensetzung.

20. Zusammensetzung nach einem der vorhergehenden Ansprüche,
**dadurch gekennzeichnet, dass** sie ein oder mehrer e organische Lösungsmittel enthält.

21. Zusammensetzung nach dem vorhergehenden Anspruch,
**dadurch gekennzeichnet, dass** das Lösungsmittel ausgewählt ist unter geradkettigen oder verzweigten niederen einwertigen Alkoholen mit 1 bis 8 Kohlenstoffatomen; Polyolen; Mono- oder Dialkylisosorbid, dessen Alkylgruppen 1 bis 5 Kohlenstoffatome aufweisen; Polyethylenglycolen mit 6 bis 80 Ethylenoxid - einheiten; Ethylenglycolethern; Propylenglycolethern; Polyolestern und Polyolethern und Estern einer Fettsäure mit einem Alkohol sowie Gemischen dieser Verbindungen.

22. Zusammensetzung nach Anspruch 20 oder 21, **dadurch gekennzeichnet, dass** die Menge des Lösungsmittels oder der Lösungsmittel im Bereich von 1 bis 50 Gew.-% liegt, bezogen auf das Gesamtgewicht der Zusammensetzung.

23. Zusammensetzung nach einem der vorhergehenden Ansprüche,
**dadurch gekennzeichnet, dass** die Ölphase 3 bis 60 Gew.-% und vorzugsweise 5 bis 30 Gew.-% ausmacht, bezogen auf das Gesamtgewicht der Zusammensetzung.

24. Zusammensetzung nach dem vorhergehenden Anspruch,
**dadurch gekennzeichnet, dass** die Ölphase mindestens ein flüchtiges Siliconöl enthält.

25. Zusammensetzung nach einem der vorhergehenden Ansprüche,
**dadurch gekennzeichnet, dass** sie ferner einen oder mehrere Hilfsstoffe enthält, die ausgewählt sind unter Gelbildungsmitteln; Hydratisierungsmitteln; Emollientien; Wirkstoffen; Mitteln gegen freie Radikale; Sequestrierungsmitteln; Antioxidationsmitteln; Konservierungsmitteln; Mitteln zum Alkalischmachen oder zum Ansäuern; Parfums; Pigmenten; filmbildenden Mitteln und Färbemitteln sowie Gemischen dieser Stoffe.

26. Zusammensetzung nach einem der vorhergehenden Ansprüche,
**dadurch gekennzeichnet, dass** sie eine kosmetische oder dermatologische Zusammensetzung darstellt.

27. Zusammensetzung nach einem der vorhergehenden Ansprüche,
**dadurch gekennzeichnet, dass** sie ein Produkt zur Pflege, zur Behandlung, zum Schutz, zur Reinigung, zum Abschminken und/oder zum Schminken von Keratinmaterialien darstellt.

28. Zusammensetzung nach dem vorhergehenden Anspruch,
**dadurch gekennzeichnet, dass** es sich bei dem Keratinmaterial um die Haut handelt.

29. Kosmetische Verwendung einer Zusammensetzung nach einem der Ansprüche 1 bis 25 als Produkt zur Pflege der Haut, der Haare, der Kopfhaut, der Wimpern, der Augenbrauen, der Nägel oder der Schleimhäute.

30. Kosmetische Verwendung einer Zusammensetzung nach einem der Ansprüche 1 bis 25 als Produkt zum Schminken.

31. Kosmetische Verwendung einer Zusammensetzung nach einem der Ansprüche 1 bis 25 als Produkt zum Sonnenschutz.

32. Kosmetische Verwendung einer Zusammensetzung nach einem der Ansprüche 1 bis 25 als Produkt zur Haarbehandlung, das gegebenenfalls ausgespült wird.

33. Kosmetische Verwendung einer Zusammensetzung nach einem der Ansprüche 1 bis 25 als Produkt zur Reinigung und/oder zum Abschminken der Haut und/oder der Augen.

34. Verfahren zur kosmetischen Behandlung eines Keratinmaterials,
**dadurch gekennzeichnet, dass** eine Zusammensetzung, wie sie in einem der Ansprüche 1 bis 25 definiert ist, auf das Keratinmaterial aufgebracht wird.

35. Verfahren nach dem vorhergehenden Anspruch, **dadurch gekennzeichnet, dass** es sich bei dem Keratinmaterial um die Haut handelt.
